# EUROPEAN PATENT APPLICATION

(11) **EP 1 618 878 A1**
(43) Date of publication of application: **25.01.2006**
(21) Application number: 04730698.0
(22) Date of filing: 30.04.2004
(51) Int. Cl.: A61K 31/198, A61K 9/08, A61K 47/02, A61P 3/00, A23L 1/305

(54) **BODY-TEMPERATURE LOWERING DEPRESSANT**

(30) Priority: 30.04.2003 JP 2003125688; 19.09.2003 JP 2003328588
(71) Applicant: Ajinomoto Co., Inc., Tokyo 104-0031 (JP)
(72) Inventor: OGAWA, Takashi, c/o AJINOMOTO CO., INC., Kawasaki-shi, Kanagawa 210-8681 (JP); KOKUBA, Yukifumi, c/o AJINOMOTO CO., INC., Kawasaki-shi, Kanagawa 210-8681 (JP); KATSURA, Toshiyuki, c/o AJINOMOTO CO., INC., Tokyo 104-0031 (JP); SATOU, Kazunori, c/o AJINOMOTO CO., INC., Kawasaki-shi, Kanagawa 210-8681 (JP)
(74) Representative: Nicholls, Kathryn Margaret
(86) International application number: PCT/JP2004/006304
(87) International publication number: WO 2004/096207

(57) **Abstract**

The present invention provides an inhibitor of decrease in body temperature which comprises at lease one kind of an amino acid(s) selected from the group consisting of valine, asparatic acid, glycine and cystein as an active substance. This inhibitor of decrease in body temperature can inhibit the decrease in body temperature caused by general anesthesia and the like, and prevent and/or improve complications accompanied with the decrease in body temperature. It is preferable that the inhibitor of decrease in body temperature contain either valine, asparatic acid, glycine or cystein in concentration of 0.01 to 10 W/V%; further contain an electrolyte(s); and the inhibitor be in the form of infusion solution preparation.

## Description

### Background of the Invention

The present invention relates to, for example, inhibitors of decrease in body temperature in the form of pharmaceutical compositions or foods, and especially those for inhibiting decrease in body temperature caused by general anesthesia during operation.

Since mechanism for controlling body temperature is inhibited by general anesthesia in patients during surgery and the like, the patients are easily affected by environmental temperature around them, and, therefore, their body temperatures (core temperatures) decrease. Due to the decrease in their body temperatures, complications develop such as chills/discomfort, shivering, tachycardia/ischemic electrocardiogram change, delay of arousal , infection of wounds, delayed wound healing, suppressed immune system, and disorder of blood coagulation.

In order to prevent such decrease in body temperature, heat retention measures are known such as aluminum heat-retention materials, circulating warming mats, warming infusion solutions and hot air warming during surgery.

However, conventional circulating warming mats have not had sufficient heat-retention effect because heat-retention area becomes localized depending on the body positions of patients. Further, though the hot air warming was an effective method, it has cost problems because expendables such as warming covers clothing the patients with are required. When body temperature decreases beyond threshold level of body temperature center, recovery of the body temperature requires long hours even if conventional heat retention measures are implemented. Further, it has been reported that high doses of standard infusion solution preparation (infusion preparation) of amino acids (total concentration of amino acids is 10 W/V % or more) are effective in inhibiting the decrease in body temperature (Eva Sellden, et al: Anesth Analg 89: 1551-1556,1999; Hiroaki Fujiwara, et al.: Geka to Taisha/Eiyou (Surgery and Metabolism/Nutrition), vol. 36, No. 4: 215-220, 2002; T. Kasai, et al.: British Journal of Anaesthesia 90: 58-61, 2003). However, an active substance of the present invention is not disclosed therein. Similarly, amino acid preparation which has the same concentration of total amino acids as that of the present invention is not disclosed therein.

### Disclosure of the Invention

The object of the present invention is to provide an inhibitor of decrease in body temperature which inhibits decrease in body temperature caused by general anesthesia and the like, and prevents and/or improves complications accompanied with the decrease in body temperature.

The further object of the present invention is to provide an inhibitor of decrease in body temperature in the form of pharmaceutical compositions or beverages and foods.

The other object of the present invention is to provide an inhibitor of decrease in body temperature which prevents and/or improves perioperative complications.

The further other object of the present invention is to provide an inhibitor of decrease in production of peripheral mononuclear blood cell TNF-α.

The further additional object of the present invention is to provide an infusion solution for intravenous administration.

In order to meet the above objects, the inventors have thoroughly studied various kinds of amino acids and found that less quantity of amino acid(s) than the quantity which has been reported until now has an action to inhibit the decrease in body temperature during general anesthesia; and some kinds of amino acids have an action to inhibit the decrease in body temperature by itself. The present invention has been completed based on these findings.

Namely, the present invention provides an inhibitor of decrease in body temperature which comprises at lease one kind of an amino acid(s) selected from the group consisting of valine, asparatic acid, glycine and cystein as an active substance.

The present invention also provides the inhibitor of decrease in body temperature which comprises a mixture of amino acids which consists of combination of at lease two kinds of amino acids selected from the group consisting of valine, asparatic acid, glycine and cystein as an active substance.

The present invention further provides an inhibitor of decrease in production of peripheral mononuclear blood cell TNF-α which comprises at lease one kind of an amino acid(s) selected from the group consisting of valine, asparatic acid, glycine and cystein as an active substance.

The present invention additionally provides an infusion solution for intravenous administration consisting of at lease one kind of an amino acid(s) selected from the group consisting of valine, asparatic acid, glycine and cystein.

### Best Mode for Carrying out the Invention

In the inhibitors of decrease in body temperature of the present invention, amino acids which can be used as an active substance are valine, asparatic acid, glycine and cystein. Valine is more preferable among them. Each of these amino acids may be contained by itself or optionally combined each other to be contained. The amino acids which are used as the active substance are preferably only valine, asparatic acid, glycine and cystein and other amino acids are preferably not contained therein. However, the other amino acids may be contained to be used, if necessary. Each of the amino acids can be used regardless of commercial products, synthesized products and other preparation methods. All of D-form, L-form and DL-form are usable and L-form is preferable. Each of the amino acids is not necessarily used as a free amino acid. They may be in the form of salts which are acceptable for pharmaceutical compositions or beverages and foods and, for example, they may be used in the forms of salts of inorganic acids and organic acids; ester body which can be hydrolyzed *in vivo*; and N-acyl derivatives. Further, they may be used in the form of peptides wherein a same kind or different kinds of amino acids are peptide-bound. Cystein may be used in the form of cystine.

As methods for administering the inhibitors of decrease in body temperature of the present invention, oral administration, intravenous administration and the like are possible, but the methods are not particularly limited to them. In case of intravenous administration, it is preferable that the inhibitors are continuously administered through peripheral veins, central veins and the like.

The doses of valine, asparatic acid, glycine and cystein which are active substances may be selected accordingly depending on administration methods, patients' symptoms or degrees thereof and kinds of dosage forms. In case of oral administration, each of the above amino acids may be administered in the range of 10mg to 10000mg per one administration. In case of intravenous administration, they may be administered in the range of about 1/20 to 1/2 of the doses of oral administration. These doses may be administered in several fractional doses, if necessary, or administered several times per one day. Meanwhile, in case of continuous administration into a vein, they are preferably administered for at lease about 2 hours in the administration speed of the range of 10mg to 1000mg/kg/hr. As the administration schedule, it is preferably administered before body temperature decreases or anesthesia is started. Further, continuous administration or continuous administration during anesthesia is also preferable.

Pharmaceutical forms of the inhibitors of decrease in body temperature of the present invention such as pharmaceutical preparations are changeable depending on the administration methods and not particularly limited. For instance, solutions, pills, capsules, granules, fine particles, powders and dispersants are thinkable. In order to prepare these dosage forms, supplements are preferably added thereto, such as suitable liquid or solid diluents, fillers, extenders, solvents, emulsifying agents, lubricants, flavor adjusting agents, flavoring agents, colorants, and buffers, all of which are pharmaceutically acceptable.

Since the inhibitors of decrease in body temperature of the present invention are preferably in the form of infusion preparation, the form of the infusion preparation is explained particularly in detail. The infusion preparation may contain any of valine, asparatic acid, glycine and cystein; or the optional combination among valine, asparatic acid, glycine and cystein. Further, amino acids except for valine, asparatic acid, glycine and cystein may not be contained. In view of possibility of their influences on the effectiveness of the amino acids which are active substances, it is rather preferable that the other amino acids are not contained therein.

In case of the infusion preparation wherein the amino acids except for valine, asparatic acid, glycine and cystein are not contained, at least one of the amino acids among valine, asparatic acid, glycine and cystein may be prepared in the range of concentration of 10 W/V% or less, preferably 0.01 to 9 W/V%, more preferably 0.03 to 6 W/V%, further more preferably 0.05 to 4 W/V% and most preferably 0.1 to 3.3W/V%

On the other hand, in case of the infusion preparation wherein the amino acids except for valine, asparatic acid, glycine and cystein are also contained, the total concentration of the amino acids is preferably less than 10 W/V%, further preferably 1 to 9 W/V%, and most preferably 1.5 to 5 W/V%. For example, in accordance with the following concentration ranges of each amino acids, it may be integrated amino acid preparation which is dispensed so that the total concentration of the amino acids is less than 10 W/V%, preferably 1 to 9 W/V%, and more preferably 1.5 to 5 W/V %.

| | |
|---|---|
| L-isoleucine | 0.05 to 1.0 (preferably 0.1 to 0.5) W/V% |
| L-leucine | 0.1 to 1.5 (preferably 0.2 to 0.8) W/V% |
| L-lysine salt | 0.1 to 1.0 (preferably 0.2 to 0.7) W/V% |
| L-methyonine | 0.02 to 0.8 (preferably 0.05 to 0.4) W/V% |
| L-phenylalanine | 0.01 to 0.8 (preferably 0.05 to 0.4) W/V% |
| L-threonine | 0.05 to 0.8 (preferably 0.1 to 0.4) W/V% |
| L-tryptophan | 0.01 to 1.0 (preferably 0.02 to 0.5) W/V% |
| L-valine | 0.01 to 2.0 (preferably 0.05 to 1.0) W/V% |
| L-alanine | 0.05 to 1.0 (preferably 0.1 to 0.5) WN% |
| L-arginine | 0.05 to 1.0 (preferably 0.1 to 0.5) W/V% |
| L-asparatic acid | 0.01 to 1.0 (preferably 0.02 to 0.5) W/V% |
| L-cystein | 0.005 to 0.5 (preferably 0.01 to 0.1) W/V% |
| L-glutamic acid | 0.005 to 0.5 (preferably 0.01 to 0.25) W/V% |
| L-histidine | 0.01 to 0.5 (preferably 0.05 to 0.3) W/V% |
| L-proline | 0.01 to 1.0 (preferably 0.05 to 0.5) W/V% |
| L-serine | 0.01 to 1.0 (preferably 0.02 to 0.5) W/V% |
| L-tyrosine | 0.005 to 0.1 (preferably 0.008 to 0.05) W/V% |
| Glycine | 0.05 to 0.1 (preferably 0.1 to 0.5) W/V% |

Meanwhile, the concentration of valine is more preferably 0.01 to 0.3 W/V% and that of glycine is more preferably 0.05 to 0.4 W/V%. Further, the concentration of cystein is preferably 0.01 to 0.05 W/V%.

The needed quantities of components such as electrolytes, sugars, pH adjusters, microelements, and vitamins, which are usually used as infusion components, may be optionally dispensed in the above infusion preparation. It is particularly preferable that the electrolytes are dispensed therein. The electrolytes herein indicate the compounds which generate ions in an aqueous solution, and they are preferably inorganic salts, organic salts and the like which generate ions such as Na⁺, K⁺ and Cl in the aqueous solution. More concretely, the electrolytes which generate the following concentration ranges of the ions are preferably dispensed therein.
- Na⁺: 20.0 to 160.0 mEq/L
- K⁺: 3.0 to 40.0 mEq/L
- Cl⁻: 15.0 to 160.0 mEq/L

The electrolytes which generate Na⁺ include sodium chloride, sodium L-lactose, sodium citrate, sodium hydrogen carbonate, sodium dihydrogen phosphate, sodium hydrogen phosphate and sodium acetate. The electrolytes which generate K⁺ include potassium chloride. The electrolytes which generate Cl include sodium chloride, potassium chloride, calcium chloride, and magnesium chloride.

The above infusion preparations are preferably prepared to be pH of 3.0 to 8.5.

As more convenient infusion preparation, valine, asparatic acid, glycine and cystein may be dispensed directly to existing infusion preparations when they are used. At that time, the inhibitors of decrease in body temperature of the present invention may be powders, granules, pills, concentrated solutions which contain needed quantities thereof, or in the form of the combination thereof. As the dispensed infusion preparations, electrolyte liquids are preferable, and an isotonic electrolyte infusion solution and a hypotonic electrolyte infusion solution, both of which are used in the perioperative period, are more preferable among them. For example, a normal saline solution, Ringer's solution, lactated Ringer's solution, acetated Ringer's solution, start fluid, dehydration supplementing fluid, preservation fluid, and postoperative recovery fluid are preferable. It may be the infusion solutions wherein sugar is dispensed therein. When the above infusion preparations are administered during the operation, they are preferably administered from 2 hours before the operation through the operation in the speed of 0.1 to 60 mL/kg/hr and preferably 10 to 40mL/kg/hr.

The infusion preparation of the inhibitors of decrease in body temperature of the present invention is preferably filled in a container. Each component may be divided into several chambers to be filled, if necessary. Since amino acids and reducing sugar may cause Maillard reaction over time when the reducing sugar is dispensed as sugar, the amino acids and the sugar are preferably isolated to be placed. For example, in an infusion solution container having two chambers, sugar/electrolytes may be filled into a first chamber and an amino acid solution(s) may be filled into a second chamber. As methods for blocking out the infusion preparation to two chambers, it is preferable to block it out with a sticker which can flake by exterior pressure at the point of use. As materials constituting a body of the infusion container, soft resin materials are preferable, which are superior in flexibility and transparency, and do not easily tear even if they fall after being preserved under low temperature.

Especially, polyolefins which are usually used as medical containers are a suitable example. Polyolefins include, for instance, polymers such as polyethylene, polypropylene, poly-1-butene and poly(4-methyl-1-pentene). The container molded by either blow, inflation or deflation molding of the resins can be used as a container body. The peripheries of two resin sheets may be deposited to form a container. In case of the form for dispensing to existing infusion preparations, it may be a container which can contain needed quantities of only needed components in the forms of powders, granules, pills, concentrated solutions or the combination thereof. A vial container may be usable. In this case, the container may be a kit product with existing infusion preparations. Meanwhile, in case of the infusion preparation containing bicarbonate ion, it is preferably produced with the method disclosed in Japanese Patent No. 3271650 and preferably filled into a gas-impermeable container, or filled into gas-permeable plastic container and then packed with a gas-impermeable second packing material.

Further, the inhibitors of decrease in body temperature of the present invention are not only able to practice in the form of the pharmaceutical compositions as mentioned above but also able to easily practice their use in beverages and foods by making them contain in the beverages and foods with reference to the embodiment of the pharmaceutical compositions.

The inhibitors of decrease in body temperature of the present invention can be used against any symptoms/pathologies as long as they intend to inhibit the decrease in body temperature and, especially, they are useful for the decrease in body temperature caused by anesthesia. The anesthesia includes general anesthesia and local anesthesia. The general anesthesia includes inhalational anesthesia with isoflurane, sevoflurane or gas which has an analgesic, calming and nyctinastic actions such as laughter gas; and intravenous anesthesia with propofol and the like. On the other hand, the local anesthesia includes spinal anesthesia (lumbar anesthesia) with tetracaine, dibucaine and the like; epidural anesthesia with lidocaine, mepivacaine, bupivacaine and the like; and so-called local anesthesia (infiltration anesthesia and surface anesthesia). The inhibitors of decrease in body temperature of the present invention are especially useful for inhibiting the decrease in body temperature by the general anesthesia during surgery among the above anesthesia.

At lease one kind of an amino acid(s) selected from the group consisting of valine, asparatic acid, glycine and cystein can introduce the usual general anesthesia without reducing the effect of an intravenous anesthesia agent (propofol) to the extent of the dose in which the present invention can effectively work.

At lease one kind of the amino acid(s) selected from the group consisting of valine, asparatic acid, glycine and cystein is useful for preventing and/or improving perioperative complications which develop during the operation and after the operation, such as chills/discomfort, shivering, tachycardia/ischemic electrocardiogram change, arousal delay, infection of wounds, delayed wound healing, suppressed immune system, and disorder of blood coagulation; and promoting recovery from operative invasion.

It is known that function of immune system is weakened by the general anesthesia during the operation, such as inhibition of production/secretion of inflammatory cytokine like TNF-α and IL-18 from peripheral mononuclear blood cells. It is also known that the immune system is inhibited by invasions such as thoracotomy and abdominal operations. It is thought to be related to prevent and/or improve perioperative complications such as infection of wounds, delayed wound healing and suppressed immune system and to promote recovery from operative invasion that at lease one kind of the amino acid(s) selected from the group consisting of valine, asparatic acid, glycine and cystein inhibits the decrease in production of TNF-α in peripheral mononuclear blood cells.

It is possible to prepare the forms of an agent for preventing and/or treating perioperative complications, an agent for promoting recovery from operative invasion, an inhibitor of decrease in production of peripheral mononuclear blood cell TNF-α and an infusion solution for intravenous administration, wherein at lease one kind of the amino acid(s) selected from the group consisting of valine, asparatic acid, glycine and cystein is contained as an active substance of the present invention, by the same methods as those of the above inhibitors of decrease in body temperature.

The present invention provides an inhibitor of decrease in body temperature which inhibits decrease in body temperature caused by general anesthesia and the like, and prevents and/or improves complications accompanied with the decrease in body temperature; and an inhibitor of decrease in body temperature which promotes recovery from operative invasion. When the inhibitors of decrease in body temperature of the present invention are used, inhibition of decrease in body temperature (core temperature) can be implemented more easily and more inexpensively compared with the conventional methods for inhibiting the decrease in body temperature, by preparation thereof added to the infusion of extracellular fluid composition which was administered in the perioperative period until now. By incorporating with the conventional methods, the inhibition of decrease in body temperature can be further effectively achieved.

Next, Examples will further illustrate the present invention.

### Example 1

### (1) Preparation of amino acid solutions

Solutions of valine, asparatic acid, glycine and cystein were prepared by dissolving them into distilled water for injection so that the concentration of each amino acids became 0.1 W/V%, 1 W/V% or 2W/V%. A mixed solution of amino acids was prepared by dissolving the amino acids of which the blending quantities were shown in Table 1 into distilled water for injection. Each solution was treated with filter sterilization before administration.

**Table 1**

| Name of amino acids | Concentration (W/V%) |
|---|---|
| L-isoleucine | 0.168 |
| L-leucine | 0.375 |
| L-lysine acetate | 0.372 |
| L-methyonine | 0.105 |
| L-phenylalanine | 0.2805 |
| L-threonine | 0.195 |
| L-tryptophan | 0.039 |
| L-valine | 0.135 |
| L-alanine | 0.186 |
| L-arginine | 0.237 |
| L-asparatic acid | 0.114 |
| L-cystein | 0.03 |
| L-glutamic acid | 0.195 |
| L-histidine | 0.18 |
| L-proline | 0.099 |
| L-serine | 0.066 |
| L-tyrosine | 0.0105 |
| Glycine | 0.321 |
| Total amino acids | 3.108 |

### (2) Measurement 1 of inhibiting effect of decrease in body temperature

A catheter was inserted into an external jugular vein of each of rats used in the evaluation in advance and a tip of the catheter was exserted from their backs and locked with heparin. The rats were recovered for a few days in the status quo. 10.5 mL/kg of each of a normal saline solution; 0.1 W/V% and 1.0 W/V% solutions of each of valine, asparatic acid, glycine and cystein; and a mixed solution of the amino acids of Table 1 were administered through the inserted catheter four times at 15-minute intervals from 60 minutes before the administration of an anesthesia agent (propofol). Then, 42 mL/kg thereof were continuously administered for 1 hour simultaneously with the start of administration of the anesthesia agent. Numbers of each group were set to 4 to 6. As for the administration of the anesthesia agent, 15 mg/kg thereof was intravenously administered through the catheter with bolus method at the beginning. At the same time, continuous administration of the agent was started in 45 mg/kg/hr. 1 hour later, administration speed was changed to 22.5 mg/kg/hr and continuous administration was conducted for total of 3 hours. As for the measurement of the body temperature, a former value was measured with a rectal thermometer before administering amino acid solutions, and values were measured at 30-minute intervals from the start of administration of the anesthesia agent until 3 hours after the end thereof.

The measurement results were calculated as variations (value before administration - value after administration) of values in 3 hours after administering the anesthesia solution to those before administration and represented with average ± standard deviation. As for a statistical test, comparison between two groups with the normal saline solution was conducted using student t-test.

The results are shown in Table 2. The inhibiting effect of decrease in body temperature (core temperature) was shown in the solutions of valine, asparatic acid, cystein and glycine and the mixed solution of amino acids.

**Table 2 NSS: normal saline solution, AA: amino acid solution**

| Conc. of Admin'ed solution | Valine | | Asparatic Acid | | Cystein | | Glycine | | Mixed amino acids | |
|---|---|---|---|---|---|---|---|---|---|---|
| | NSS | AA | NSS | AA | NSS | AA | NSS | AA | NSS | AA |
| 0.1% | -7.0 | -4.8 | -6.9 | -6.4 | -7.3 | -6.3 | -7.5 | -6.4 | | |
| | ±0.5 | ±1.4 | ±1.1 | ±1.0 | ±1.1 | ±0.8 | ±0.2 | ±1.5 | | |
| 1% | -7.0 | -4.0 | -6.9 | -4.5 | -7.3 | -4.8 | -7.9 | -6.1 | -6.6 | -5.5 |
| | ±0.6 | ±0.7 | ±0.7 | ±0.9 | ±1.4 | ±1.0 | ±0.7 | ±0.9 | ±1.1 | ±1.3 |
| 3% | | | | | | | | | -6.6 | -4.0 |
| | | | | | | | | | ±1.1 | ±0.9 |

| | | | | | | | | | | |
|---|---|---|---|---|---|---|---|---|---|---|
| (average ± standard deviation, underline P < 0.01, unit: °C) | | | | | | | | | | |

### (3) Measurement 2 of inhibiting effect of decrease in body temperature

Examination was conducted on whether differences of the inhibiting effect of decrease in body temperature of valine were shown depending on difference of timings of administration (following A to D). A catheter was inserted into an outward jugular vein of each of rats (7 to 8 weeks age) used in the evaluation in advance and a tip of the catheter was exserted from their backs and locked with heparin. The rats were recovered for a few days in the status quo. Valine was administered through the catheter with the following four methods:
A: for 2 hours before the start of administration of propofol
B: for 1 hour before the start of administration of propofol + 1 hour after the start of administration of propofol
C: for 2 hours simultaneously with the start of administration of propofol
D: for 2 hours starting from 1 hour after the start of administration of propofol. 105 mg/kg of valine containing 10.5 mL/kg of 1% valine solution was administered to Group A eight times at 15-minute intervals. 105 mg/kg of valine containing 10.5 mL/kg of 1% valine solution was administered to Group B four times at 15-minute intervals and then continuously administered for 1 hour in 420 mg/kg/hr containing 42 mL/kg of 1% valine solution. Valine was continuously administered to Groups C and D for 2 hours in 420 mg/kg/hr containing 42 mL/kg of 1% valine solution. Each group (n = 8 to 9) was administered the same total dose of valine. 15 mg/kg of a propofol anesthesia agent was administered through the catheter at the beginning and, after induction of anesthesia, it was continuously administered for 3 hours in 33.75 mg/kg/hr.

As a result, the decrease in body temperature from the time before administration until the end of the anesthesia was 6.1 ± 1.9°C for Group A; 4.3 ± 1.2°C for Group B; 6.1 ± 1.4°C for Group C; and 6.9 ± 1.0°C for Group D. The decrease range in body temperature of Group B was least, then those of Groups A and C were about the same, and the inhibiting effect of decrease in body temperature was not shown in Group D. Further, the increase in body temperature from the end of administration of the anesthesia until 1 hour after the end of administration of the anesthesia was highest in Group B (2.0 ± 1.2°C) and Group C (2.0 ± 0.9°C), then Group A (1.1 ± 0.5°C) and Group D (1.0 ± 0.6°C). From the above, valine had a stronger inhibiting activity of the decrease in body temperature when it was administered before the start of administration of the propofol anesthesia agent or simultaneously with administration of the anesthesia agent. The increase in body temperature after the end of the administration of the anesthesia agent was prominently shown when valine was administered before or after administration of the anesthesia agent, or simultaneously with administration of the anesthesia agent. Thus, it is preferable that valine is administered before administration of the anesthesia agent and during administration of the anesthesia agent.

### (4) Effect on recovery from operative invasion

A catheter was inserted into an external jugular vein of each of rats (7 weeks age) used in the evaluation in advance and a tip of the catheter was exserted from their backs and locked with heparin. The rats were recovered for a few days in the status quo. 105 mg/kg of valine (5.25 mL/kg of 2 W/V% of valine solution) was administered through the catheter to a valine-administered group four times at 15-minute intervals from 60 minutes before the start of administration of a propofol anesthesia agent. Then, valine was continuously administered for 5 hours in 420 mg/kg/hr (21 mL/kg of 2 W/V% valine solution) simultaneously with the start of administration of the propofol anesthesia agent. A normal saline solution was administered instead of a valine solution to an objective group. As for the administration of the propofol anesthesia agent, 15 mg/kg thereof was intravenously administered through the catheter with bolus method at the beginning. Soon after that, the agent was started to be continuously administered in 45 mg/kg/hr. 1 hour later, the administration speed was changed to 33.75 mg/kg/hr and continuous administration was conducted for 5 hours. Numbers of each group were set to 8. As for surgical invasion, abdominals of the rats were opened after the bolus anesthesia, their appendixes were exteriorized from their abdominal cavities and exposed in the air for 30 minutes. Then, their appendixes were put back to their abdominal cavities and sutured. As for the measurement of the body temperatures, a former value was measured with a rectal thermometer before administering the amino acids, and the value was measured again in 3 hours after the administration of propofol. The results were shown as the decreased temperature to Pre value. Survival rates were calculated from number of survivors in 24 hours from the start of the experiment.

As a result, while the decrease in body temperature of the objective group was 9.02±0.66°C (n = 6, two were dead before the measurement in 3 hours after the administration), that of the valine-administered group was 7.83±0.87°C (n = 8) and the decrease in body temperature was inhibited by 1.19 °C . The survival rates after one day was 87.5% for the valine-administered group compared with 12.5% for the objective group and, therefore, the survival rate was improved by the administration of valine.

### (5) Effect on production of peripheral mononuclear blood cell TNF-α

The examination was practiced with three groups of a normal group, a valine-administered group and an objective group. A catheter was inserted into an external jugular vein of each of rats (7 to 8 weeks age) used in the evaluation in advance and a tip of the catheter was exserted from their backs and locked with heparin. The rats were recovered for a few days in the status quo. 105 mg/kg of valine (5.25 mL/kg of 2 W/V% of valine solution) was administered through the inserted catheter to the valine-administered group (n = 6) four times at 15-minute intervals from 60 minutes before the start of administration of a propofol anesthesia agent. Then, valine was continuously administered for 1 hour in 420 mg/kg/hr (21 mL/kg of 2 W/V% valine solution) simultaneously with the start of administration of the propofol anesthesia agent. A normal saline solution was administered instead of valine to the objective group (n = 6). 15mg/kg of the propofol anesthesia agent was introduced to be administered through the catheter at the beginning, and continuously administered for 3 hours in 33.75mg/kg/hr. The celiotomy invasion was conducted by opening the abodominals of the rats simultaneously with the administration of the anesthesia, exteriorizing their appendixes from the abdominal cavities, putting them back again at once and suturing the abdominals. The catheter was not inserted to the normal group. Neither test solution nor propofol was administered to the normal group. Concurrently with the end of the administration of anesthesia, blood were taken from aorta abdominalis, peripheral mononuclear blood cells (PBMC) were separated/purified with Nycoprep 1.077A by hydrometry. PBMC was incubated on RPMI1640 medium containing 10% FCS under the existence of 1 µg/mL of lipopolysaccharide (LPS) for 24 hours (37°C, 95% of air, 5% of CO₂). Culture supernatant was collected in 24 hours after the start of the incubation and cryopreserved until the measurement of TNF-α. The measurement of TNF-α was conducted by ELISA.

As a result, the production quantity of TNF-α of the objective group (619.3±367.4pg/1 × 10⁶cells) was lower compared with that of the normal group (1141.7±L288.6pg/1 × 10⁶cells). However, the production quantity of the valine-administered group (1389.8 ± 415.8pg/l × 10⁶cells) was significantly higher (p<0.05) compared with that of the objective group and about the same as that of the normal group. The body temperatures at the end of the administration of the anesthesia were 31.2 ± 0.8°C for the valine-administered group and 30.0 ± 1.1°C for the objective group and, therefore, the valine-administered group indicated a higher value compared with the objective group.

### Example 2

The amino acids and electrolytes having blending quantities of Table 3 were prepared by being dissolved into distilled water for injection, filtered and sterilized (105°C, 25 minutes). The concentrations of each ions at that time were Na⁺: 130mEq/L, K⁺: 4mEq/L, Ca²⁺: 3mEq/L, Cl⁻: 109mEq/L, and L-Lactate: 28mEq/L. pH was 6.21 to 6.22.

**Table 3**

| Name of substances | Concentration (W/V%) |
|---|---|
| L-valine | 3.3 |
| sodium chloride | 0.60 |
| potassium chloride | 0.03 |
| calcium chloride (dihydrate) | 0.02 |
| sodium L-lactate | 0.31 |
| Total amino acids | 3.3 |

### Example 3

The amino acids and electrolytes having blending quantities of Table 4 were prepared by being dissolved into distilled water for injection, filtered and sterilized. The concentrations of each ions at that time were Na⁺: 90mEq/L, Cl⁻: 70mEq/L, and L-Lactate⁻: 20mEq/L. pH was 6.13 to 6.17.

**Table 4**

| Name of substances | Concentration (W/V%) |
|---|---|
| L-valine | 3.3 |
| sodium chloride | 0.414 |
| sodium L-lactate | 0.224 |
| glucose | 2.6 |
| Total amino acids | 3.3 |

### Example 4

The amino acids and electrolytes having blending quantities of Table 5 are prepared by being dissolved into distilled water for injection, filtered and sterilized. The concentrations of each ions at this time are Na⁺: 84mEq/L, K⁺: 20mEq/L, Cl⁻: 66mEq/L, and L-Lactate⁻: 20mEq/L. pH is 3.5 to 6.5.

**Table 5**

| Name of substances | Concentration (W/V%) |
|---|---|
| L-valine | 3.3 |
| sodium chloride | 0.270 |
| potassium chloride | 0.149 |
| sodium L-lactate | 0.224 |
| sodium dihydrogen phosphate | 0.031 |
| sodium hydrogen phosphate | 0.287 |
| glucose | 3.2 |
| Total amino acids | 3.3 |

### Example 5

The amino acids and electrolytes having blending quantities of Table 6 are prepared by being dissolved into distilled water for injection, filtered and sterilized. The concentrations of each ions at this time are Na⁺: 35mEq/L, K⁺: 20mEq/L, Cl⁻: 35mEq/L, and L-Lactate: 20mEq/L. pH is 3.5 to 6.5.

**Table 6**

| Name of substances | Concentration (W/V%) |
|---|---|
| L-valine | 3.3 |
| sodium chloride | 0.090 |
| potassium chloride | 0.149 |
| sodium L-lactate | 0.224 |
| glucose | 4.3 |
| Total amino acids | 3.3 |

### Example 6

The amino acids and electrolytes having blending quantities of Table 7 are prepared by being dissolved into distilled water for injection, filtered and sterilized. The concentrations of each ions at this time are Na⁺: 30mEq/L, Cl⁻: 20mEq/L, and L-Lactate⁻: 10mEq/L. pH is 3.5 to 6.5.

**Table 7**

| Name of substances | Concentration (W/V%) |
|---|---|
| L-valine | 3.3 |
| sodium chloride | 0.117 |
| sodium L-lactate | 0.112 |
| glucose | 4.3 |
| Total amino acids | 3.3 |

### Example 7

The amino acids and electrolytes having blending quantities of Table 8 were prepared by being dissolved into distilled water for injection, filtered and sterilized. The concenrations of each ions at that time were Na⁺: 90mEq/L, Cl⁻: 90mEq/L, and L-Lactate⁻: 20mEq/L. pH was 5.27.

**Table 8**

| Name of substances | Concentration (W/V%) |
|---|---|
| L-valine | 1.0 |
| sodium chloride | 0.414 |
| sodium L-lactate | 0.224 |
| Total amino acids | 1.0 |

### Example 8

The amino acids and electrolytes having blending quantities of Table 9 were prepared by being dissolved into distilled water for injection, filtered and sterilized. The concentrations of each ions at that time were Na⁺: 90mEq/L, Cl⁻: 90mEq/L, and L-Lactate⁻: 20mEq/L. pH was 5.37.

**Table 9**

| Name of substances | Concentration (W/V%) |
|---|---|
| L-valine | 2.7 |
| sodium chloride | 0.414 |
| sodium L-lactate | 0.224 |
| Total amino acids | 2.7 |

### Example 9

The amino acids and electrolytes having blending quantities of Table 10 were prepared by being dissolved into distilled water for injection, filtered and sterilized. The concentrations of each ions at that time were Na⁺: 90mEq/L, Cl⁻: 90mEq/L, and L-Lactate⁻: 20mEq/L. pH was 5.44.

**Table 10**

| Name of substances | Concentration (W/V%) |
|---|---|
| L-valine | 4.3 |
| sodium chloride | 0.414 |
| sodium L-lactate | 0.224 |
| Total amino acids | 4.3 |

### Example 10

The amino acid having a blending quantity of Table 11 was prepared by being dissolved into distilled water for injection, filtered and sterilized. pH was 5.71 at that time.

**Table 11**

| Name of substances | Concentration (W/V%) |
|---|---|
| L-valine | 3.3 |
| Total amino acids | 3.3 |

### Example 11

The amino acid having a blending quantity of Table 12 was prepared by being dissolved into distilled water for injection, filtered and sterilized. pH was 5.92 at that time.

**Table 12**

| Name of substances | Concentration (W/V%) |
|---|---|
| L-valine | 5.5 |
| Total amino acids | 5.5 |

### Example 12

The amino acids and electrolytes having blending quantities of Table 13 are prepared by being dissolved into distilled water for injection, filtered and sterilized (105°C, 25 minutes). The concentrations of each ions at this time are Na⁺: 135mEq/L, K⁺: 4mEq/L, Ca²⁺: 3mEq/L, Mg²⁺: ImEq/L, Cl : 113mEq/L, HCO₃: 25mEq/L, and Citrate: 5mEq/L. pH is 6.5 to 7.8.

**Table 13**

| Name of substances | Concentration (W/V%) |
|---|---|
| L-valine | 1.0 |
| sodium chloride | 0.614 |
| potassium chloride | 0.03 |
| calcium chloride | 0.022 |
| magnesium chloride | 0.0102 |
| sodium citrate | 0.049 |
| sodium hydrogen carbonate | 0.21 |
| Total amino acids | 1.0 |

### Example 13

The amino acids and electrolytes having blending quantities of Table 14 were prepared by being dissolved into distilled water for injection, filtered and sterilized (105°C, 25 minutes). The concentrations of each ions at this time were Na⁺: 135mEq/L, K⁺: 4mEq/L, Ca²⁺: 3mEq/L, Mg²⁺: 1mEq/L, Cl: 113mEq/L, HCO₃⁻: 25mEq/L, and Citrate: 5mEq/L. pH was 6.6.

**Table 14**

| Name of substances | Concentration (W/V%) |
|---|---|
| L-valine | 2.0 |
| sodium chloride | 0.614 |
| potassium chloride | 0.03 |
| calcium chloride | 0.022 |
| magnesium chloride | 0.0102 |
| sodium citrate | 0.049 |
| sodium hydrogen carbonate | 0.21 |
| Total amino acids | 2.0 |

### Example 14

The amino acids and electrolytes having blending quantities of Table 15 are prepared by being dissolved into distilled water for injection, filtered and sterilized (105°C, 25 minutes). The concentrations of each ions at this time are Na⁺: 135mEq/L, K⁺: 4mEq/L, Ca²⁺: 3mEq/L, Mg²⁺: 1mEq/L, Cl : 113mEq/L, HCO₃⁻: 25mEq/L, and Citrate: 5mEq/L. pH is 6.5 to 7.8.

**Table 15**

| Name of substances | Concentration (W/V%) |
|---|---|
| L-valine | 3.3 |
| sodium chloride | 0.614 |
| potassium chloride | 0.03 |
| calcium chloride | 0.022 |
| magnesium chloride | 0.0102 |
| sodium citrate | 0.049 |
| sodium hydrogen carbonate | 0.21 |
| Total amino acids | 3.3 |

### Example 15

The amino acids and electrolytes having blending quantities of Table 16 were prepared by being dissolved into distilled water for injection, filtered and sterilized (105°C, 25 minutes). The concentrations of each ions at that time were Na⁺:154mEq/L and Cl⁻: 154mEq/L, and pH was 5.80.

**Table 16**

| Name of substances | Concentration (W/V%) |
|---|---|
| L-valine | 1.0 |
| sodium chloride | 0.9 |
| Total amino acids | 1.0 |

### Example 16

The amino acids and electrolytes having blending quantities of Table 17 were prepared by being dissolved into distilled water for injection, filtered and sterilized (105°C, 25 minutes). The concentrations of each ions at that time were Na⁺: 154mEq/L and Cl : 154mEq/L, and pH was 5.56.

**Table 17**

| Name of substances | Concentration (W/V%) |
|---|---|
| L-valine | 3.3 |
| sodium chloride | 0.9 |
| Total amino acids | 3.3 |

### Example 17

The amino acids and electrolytes having blending quantities of Table 18 were prepared by being dissolved into distilled water for injection, filtered and sterilized. The concentrations of each ions at that time were Na⁺: 35mEq/L, K⁺: 20mEq/L, Cl⁻: 35mEq/L, and L-Lactate: 20mEq/L. pH was 5.34.

**Table 18**

| Name of substances | Concentration (W/V%) |
|---|---|
| L-valine | 2.1 |
| sodium chloride | 0.09 |
| potassium chloride | 0.149 |
| sodium L-lactate | 0.224 |
| Total amino acids | 2.1 |

### Example 18

The amino acids and electrolytes having blending quantities of Table 19 were prepared by being dissolved into distilled water for injection, filtered and sterilized. The concentrations of each ions at that time were Na^{+:} 35mEq/L, K⁺: 20mEq/L, Cl: 35mEq/L, and L-Lactate: 20mEq/L. pH was 5.41.

**Table 19**

| Name of substances | Concentration (W/V%) |
|---|---|
| L-valine | 3.7 |
| sodium chloride | 0.09 |
| potassium chloride | 0.149 |
| sodium L-lactate | 0.224 |
| Total amino acids | 3.7 |

### Example 19

The amino acids and electrolytes having blending quantities of Table 20 were prepared by being dissolved into distilled water for injection, filtered and sterilized. The concentrations of each ions at that time were Na^{+:} 35mEq/L, K⁺: 20mEq/L, Cl⁻: 35mEq/L, and L-Lactate⁻: 20mEq/L. pH was 5.49.

**Table 20**

| Name of substances | Concentration (W/V%) |
|---|---|
| L-valine | 5.4 |
| sodium chloride | 0.09 |
| potassium chloride | 0.149 |
| sodium L-lactate | 0.224 |
| Total amino acids | 5.4 |

### Example 20

The amino acids and electrolytes having blending quantities of Table 21 are prepared by being dissolved into distilled water for injection, filtered and sterilized. The concentrations of each ions at this time are Na⁺: 130mEq/L, K⁺: 4mEq/L, Ca²⁺: 3mEq/L, Cl⁻: 109mEq/L, and L-Lactate⁻: 28mEq/L. pH is 6.0 to 7.5.

**Table 21**

| Name of substances | Concentration (W/V%) |
|---|---|
| L-valine | 1.0 |
| sodium chloride | 0.60 |
| potassium chloride | 0.03 |
| calcium chloride (dihydrate) | 0.02 |
| sodium L-lactate | 0.31 |
| Total amino acids | 1.0 |

### Example 21

The amino acids and electrolytes having blending quantities of Table 22 are prepared by being dissolved into distilled water for injection, filtered and sterilized (105°C, 25 minutes). The concentrations of each ions at this time are Na⁺: 130mEq/L, K⁺: 4mEq/L, Ca²⁺: 3mEq/L, Cl: 109mEq/L, and Acetate : 28mEq/L. pH is 6.5 to 7.5.

**Table 22**

| Name of substances | Concentration (W/V%) |
|---|---|
| L-valine | 1.0 |
| sodium chloride | 0.6 |
| potassium chloride | 0.03 |
| calcium chloride | 0.02 |
| sodium acetate | 0.38 |
| Total amino acids | 1.0 |

### Example 22

The amino acids and electrolytes having blending quantities of Table 23 were prepared by being dissolved into distilled water for injection, filtered and sterilized (105°C, 25 minutes). The concentrations of each ions at that time were Na⁺: 130mEq/L, K⁺: 4mEq/L, Ca^{2+:} 3mEq/L, Cl⁻: 109mEq/L, and Acetate⁻: 28mEq/L. pH was 6.68.

**Table23**

| Name of substances | Concentration (W/V%) |
|---|---|
| L-valine | 2.0 |
| sodium chloride | 0.6 |
| potassium chloride | 0.03 |
| calcium chloride | 0.02 |
| sodium acetate | 0.38 |
| Total amino acids | 2.0 |

### Example 23

The amino acids and electrolytes having blending quantities of Table 24 are prepared by being dissolved into distilled water for injection, filtered and sterilized (105°C, 25 minutes). The concentrations of each ions at this time are Na⁺: 130mEq/L, K⁺: 4mEq/L, Ca²⁺: 3mEq/L, Cl: 109mEq/L, and Acetate: 28mEq/L. pH is 6.5 to 7.5.

**Table 24**

| Name of substances | Concentration (W/V%) |
|---|---|
| L-valine | 3.3 |
| sodium chloride | 0.6 |
| potassium chloride | 0.03 |
| calcium chloride | 0.02 |
| sodium acetate | 0.38 |
| Total amino acids | 3.3 |

### Example 24

### (1) Preparation of a sugar/electrolyte solution

In accordance with blending quantities of Table 25, sodium chloride, sodium L-lactate and glucose are dissolved into distilled water for injection, and a sugar/electrolyte solution is prepared.

**Table 25**

| Name of substances | Concentration (W/V%) |
|---|---|
| sodium chloride | 0.591 |
| sodium L-lactate | 0.320 |
| glucose | 3.7 |

### (2) Preparation of an amino acid solution

L-valine is dissolved into distilled water for injection so that it becomes 5 W/V% and an amino acid solution is prepared.

### (3) Filling to plastic containers, sterilization and packaging thereof

Aseptic filtration is conducted to both of the prepared sugar/electrolyte solution and the amino acid solution. 340mL of the sugar/electrolyte solution is filled in a first chamber of a plastic container divided with a partition wall (doublebag). After sealing the first chamber, 660mL of the amino acid solution is filled in a second chamber to each plastic containers and sealed. High-pressure steam sterilization is conducted to the plastic container wherein the both solutions are filled and sealed. After cooling down the container, it is packed by packing materials with a deoxidant.

## Claims

1. An inhibitor of decrease in body temperature which comprises at lease one kind of an amino acid(s) selected from the group consisting of valine, asparatic acid, glycine and cystein as an active substance.

2. The inhibitor of decrease in body temperature according to claim 1, wherein the amino acid is valine.

3. An inhibitor of decrease in body temperature which comprises a mixture of amino acids which consists of a combination of at lease two kinds of amino acids selected from the group consisting of valine, asparatic acid, glycine and cystein as an active substance.

4. The inhibitor of decrease in body temperature according to claim 3, wherein the mixture of the amino acids contains valine.

5. The inhibitor of decrease in body temperature according to any one of claims 1 to 4, which is in the form of pharmaceutical composition.

6. The inhibitor of decrease in body temperature according to claim 5, wherein the pharmaceutical composition is in the form of infusion preparation.

7. The inhibitor of decrease in body temperature according to claim 6, wherein the concentration of either valine, asparatic acid, glycine or cystein is 0.01 to 10 W/V%.

8. The inhibitor of decrease in body temperature according to claim 6, wherein the concentration of valine is 0.01 to 10 W/V%.

9. The inhibitor of decrease in body temperature according to any one of claims 6 to 8, wherein the total concentration of amino acids is less than 10 W/V%.

10. The inhibitor of decrease in body temperature according to any one of claims 5 to 9, which further comprises an electrolyte(s).

11. The inhibitor of decrease in body temperature according to any one of claims 5 to 10, which is dispensed in infusion preparation at the time of use.

12. The inhibitor of decrease in body temperature according to any one of claims 1 to 4, which is in the form of beverages or foods.

13. The inhibitor of decrease in body temperature according to any one of claims 1 to 12, which does not contain amino acids except for valine, asparatic acid, glycine and cystein.

14. The inhibitor of decrease in body temperature according to any one of claims 1 to 13, which prevents and/or improves perioperative complications.

15. The inhibitor of decrease in body temperature according to any one of claims 1 to 13, which promotes recovery from operative invasion.

16. The inhibitor of decrease in body temperature according to any one of claims 1 to 13, which inhibits decrease in production of TNF-α in peripheral mononuclear blood cells.

17. The inhibitor of decrease in body temperature according to any one of claims 1 to 13, which is used for administration before decrease in body temperature or the start of anesthesia.

18. An agent for preventing and/or treating perioperative complications which comprises at lease one kind of an amino acid(s) selected from the group consisting of valine, asparatic acid, glycine and cystein as an active substance.

19. An agent for promoting recovery from operative invasion which comprises at lease one kind of an amino acid(s) selected from the group consisting of valine, asparatic acid, glycine and cystein as an active substance.

20. An inhibitor of decrease in production of peripheral mononuclear blood cell TNF-α which comprises at lease one kind of an amino acid(s) selected from the group consisting of valine, asparatic acid, glycine and cystein as an active substance.

21. An infusion solution for intravenous administration consisting of at lease one kind of an amino acid(s) selected from the group consisting of valine, asparatic acid, glycine and cystein.

22. The infusion solution for intravenous administration according to claim 21, which further contains an electrolyte(s).

23. The infusion solution for intravenous administration according to any one of claims 21 and 22, which is used for perioperative patients.
